# EUROPEAN PATENT APPLICATION

(11) **EP 0 941 714 A1**
(43) Date of publication of application: **15.09.1999**
(21) Application number: 98301711.2
(22) Date of filing: 09.03.1998
(51) Int. Cl.: A61F 2/06, A61F 2/00, A61L 31/00, A61F 2/04

(54) **Urological stent**

(71) Applicant: BIOCOMPATIBLES LIMITED, Farnham, Surrey GU9 8QL (GB)
(72) Inventor: Woodroffe, Matthew John, Turk Street, Alton, Hants, GU34 1DT (GB); Moore, David, Elmesthorpe, Leicester LE9 7SA (GB); Russell, Jeremy Colin, c/o Biocompatibles Ltd, Waydon Lane, Farnham, Surrey GU9 8QL (GB); Sutton, Terence Michael, c/o Biocompatibles Ltd, Weydon Lane, Farnham, Surrey GU9 8QL (GB)
(74) Representative: Haley, Stephen

(57) **Abstract**

A urological stent (1) comprises a radially compressible tubular member (2) and bladder wall engaging means (3) deployable in use, to engage with the bladder wall of a patient and retain the stent in position, the retaining means (7) being attached to one end of the tubular member.

## Description

The present invention relates to urological stents.

There are several designs of stent available for many different applications. Three basic categories of stent are heat-expandable devices, balloon-expandable devices, and self-expanding devices. The present invention is concerned with a self-expanding stent which is of a design which is of particular benefit in urological applications.

According to the present invention there is provided a urological stent comprising:
a radially compressible tubular member; and
bladder wall engaging means, deployable in use, to engage with the bladder wall of a patient and retain the stent in position, the retaining means being attached to one end of the tubular member and provided by a plurality of engaging members biased to expand, upon deployment, and engage with the bladder wall of a patient.

The members may be formed from a single folded wire. The members may be formed from a single etched metal sheet which is either planar or conical. The members may be encased in an elastomer.

The tubular member may be formed from two sets of mutually counter-rotating helical filaments which are braided together to define the member. Again, the filaments may be encased in an elastomer.

The bladder engaging means and the tubular member may be attached to one another by an elastomer.

The tubular member may be axially tapered toward the point at which it is attached to the bladder engaging means.

The stent may be encased or coated with a biocompatible material. Appropriate biocompatible materials are described in WO-A-93/01221, WO-A-94/16748, WO-A-94/16749 and WO-A-93/15775.

The elastomer coating that may be provided over the tubular member and/or the bladder engaging means may be silicone rubber.

The stent of the invention is particularly useful in the treatment of prostatic hyperplasia. The provision of bladder wall engaging means ensures that the stent does not move once inserted into a patient. Where appropriate, this ensures that the proximal end of the stent does not contact the external sphincter and cause incontinence.

The bladder wall engaging means is preferably arranged so that it can be compressed down to a diameter similar to that of the tubular member for ease of deployment. The engaging means is preferably normally biased to remain in its deployed, expanded, state.

The provision of a stent which can be compressed and then expands upon placement, together with the provision of self-expanding bladder wall engaging means, provides a device which conforms well to the bladder wall of a patient and ensures that even inaccurate placement of the stent does not adversely effect its operation or inability to move once deployed. Furthermore, provision of an elastomer connection between the tubular member and bladder wall engaging means allows the two components to flex and settle independently of one another, allowing greater conformity of the stent to the bladder walls and the prostatic urethra.

One example of the present invention will now be described with reference to the accompanying drawings, in which:

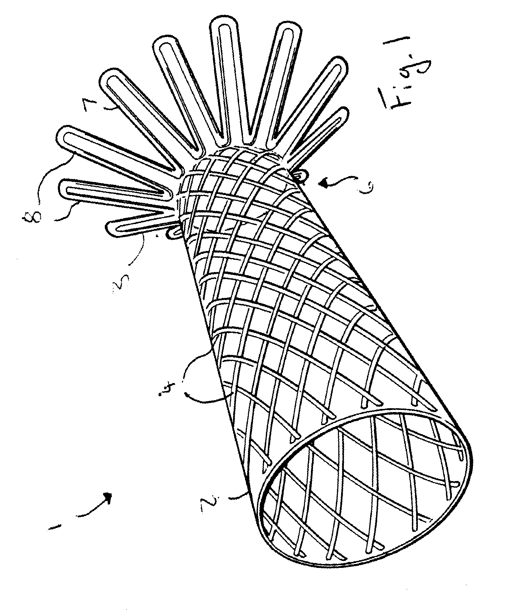

Figure 1 is a side perspective view of a stent according to the present invention.

Referring to figure 1, a stent 1 according to the present invention comprises a tubular member 2 and bladder wall engaging means 3. Figure 1 shows the stent in its expanded condition.

In use the stent is compressed radially with the bladder wall engaging means 3 being compressed into a cylindrical shape with a diameter similar to that of the tubular member 2, which is also compressed for delivery.

Upon delivery of the stent 1 to the urethra of a patient the stent 1 is allowed to expand, the tubular member 2 expanding and engaging with the walls of the urethra. The delivery of the stent is such that the bladder wall engaging means 3 is positioned within the bladder of a patient and expands engaging with the wall of the bladder and drawing the tubular member toward the bladder. The tubular member is, in this example, tapered toward the end at which it is attached to the bladder wall engaging means 3, stopping excessive migration of the tubular member 2 towards the bladder and helping to retain the stent 1 firmly in position.

As can be seen from figure 1, the tubular member 2 is formed from braided counter rotating helical filaments 4 which are encased completely, providing a continuous sheath from a proximal end 5 of the tubular member 2 to the point 6 at which it connects with the bladder wall engaging means 3. The proximal end of the tubular member 2 may be dipped in the same encasing material to ensure that none of the filaments 4 protrude from the end of the stent 1. An elastomer can be used as the encasing material. A suitable elastomer is silicone rubber, although other materials may be used. Any taper in the tubular member 2 may be provided by heat treatment of the braided filaments on a conical mandrel or by compression provided in the encasing material.

It is possible for the encasing material to be provided so that holes are left between the filaments whilst ensuring that the filaments still remain totally encased. Such holes are particularly useful if the stent is not intended to be removed from the patient.

In this example the bladder wall engaging means 3 is formed from a single folded wire 7 which is joined at its two ends to form plural members 8 (for example 6 or 12) which are biased into an expanded position. In this example the wire 7 is bent so that the sides of each member 8 are parallel to one another, but this is not essential. This improves the ease with which the members can be urged together into a compressed state for delivery. Again, the wire 7 engaging means 3 is encased completely in a suitable material, such as silicone rubber.

The filaments and the wire forming the bladder wall engaging means 3 are of a metal suitable for surgical implantation.

The bladder wall engaging means 3 is preferably coated with or encapsulated in a biocompatible material that prevents encrustation occurring due to its presence in the bladder.

## Claims

1. A urological stent comprising:
a radially compressible tubular member; and
bladder wall engaging means, deployable in use, to engage with the bladder wall of a patient and retain the stent in position, the retaining means being attached to one end of the tubular member wherein the engaging means is provided by plural engaging members biased to expand, upon deployment, and engage with the bladder wall of a patient.

2. A stent according to claim 1, wherein the members are formed from a single folded wire.

3. A stent according to claim 1, wherein the members are formed from a sheet of etched metal.

4. A stent according to any of claims 1 to 3, wherein the tubular member is formed from two sets of mutually counter-rotating helical filaments which are braided together to define the member.

5. A stent according to any of the preceding claims, wherein the bladder wall engaging means is encased in an elastomer.

6. A stent according to any of the preceding claims, wherein the tubular member is encased in an elastomer.

7. A stent according to any of the preceding claims, wherein the bladder engaging means and the tubular member are attached to one another by an elastomer.

8. A stent according to any of claims 5 to 7, wherein the elastomer is silicone rubber.

9. A stent according to any of the preceding claims, wherein the tubular member is axially tapered toward the point at which it is attached to the bladder engaging means.

10. A stent according to any of the preceding claims, wherein the stent is coated with a biocompatible material.

11. A stent according to any of the preceding claims, wherein the bladder wall engaging means is arranged so that it can be compressed down to a diameter similar to that of the tubular member.

12. A stent according to any of the preceding claims, wherein the number of members is in the range of 2 to 24.
